(19) Europäisches Patentamt
European Patent Office
Office européen des brevets

(11) Veröffentlichungsnummer: **0 386 572 B1**

(12) **EUROPÄISCHE PATENTSCHRIFT**

(45) Veröffentlichungstag der Patentschrift: **22.12.93**

(51) Int. Cl.⁵: **C07C 69/753**, C07C 67/00, A01N 53/00

(21) Anmeldenummer: **90103673.1**

(22) Anmeldetag: **26.02.90**

Die Akte enthält technische Angaben, die nach dem Eingang der Anmeldung eingereicht wurden und die nicht in dieser Patentschrift enthalten sind.

# (54) Substituierte 2-Phenyl-alk(en)-1-yl-cyclopropancarbonsäurebenzylester, Verfahren zu ihrer Herstellung und ihre Verwendung zur Bekämpfung von Schädlingen.

(30) Priorität: **04.03.89 DE 3907069**

(43) Veröffentlichungstag der Anmeldung:
**12.09.90 Patentblatt 90/37**

(45) Bekanntmachung des Hinweises auf die Patenterteilung:
**22.12.93 Patentblatt 93/51**

(84) Benannte Vertragsstaaten:
**BE CH DE DK ES FR GB IT LI NL**

(56) Entgegenhaltungen:
**EP-A- 0 143 152**
**EP-B- 0 049 977**
**US-A- 4 536 591**

(73) Patentinhaber: **BASF Aktiengesellschaft**
**Carl-Bosch-Strasse 38**
**D-67063 Ludwigshafen(DE)**

(72) Erfinder: **Zombik, Winfried, Dr.**
**Kallstadter Strasse 4**
**D-6804 Ilvesheim(DE)**
Erfinder: **Theobald, Hans, Dr.**
**Oueichstrasse 6**
**D-6703 Limburgerhof(DE)**
Erfinder: **Wolf, Bernd, Dr.**
**Hallbergstrasse 4**
**D-6701 Fussgoenheim(DE)**
Erfinder: **Schuster, Ludwig, Dr.**
**Weinheimer Strasse 44**
**D-6703 Limburgerhof(DE)**
Erfinder: **Hofmeister, Peter, Dr.**
**Bernard-Humblot-Strasse 12**
**D-6730 Neustadt(DE)**
Erfinder: **Kuenast, Christoph, Dr.**
**Salierstrasse 2**
**D-6701 Otterstadt(DE)**

EP 0 386 572 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 2-Phenyl-alk(en)-1-yl-cyclopropancarbonsäurebenzylester der allgemeinen Formeln Ia und Ib

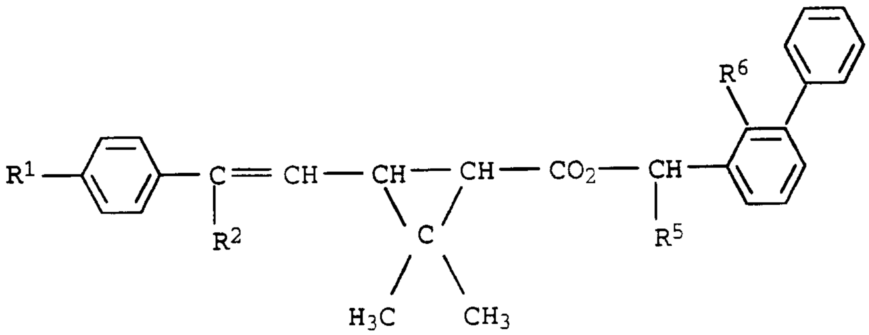

Ia

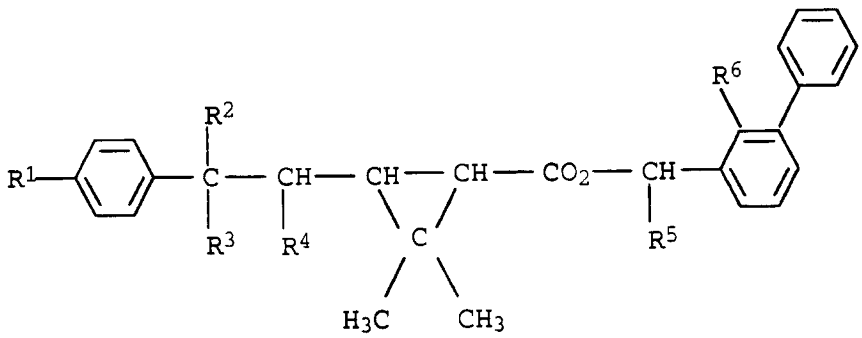

Ib

in denen die Substituenten die folgende Bedeutung haben:

$R^1$ Fluor, Chlor, Methyl, t.-Butyl, Trifluormethyl, Methoxy oder Trifluormethoxy;
$R^2$ Chlor oder Brom;
$R^3$ und $R^4$ gleichzeitig Chlor oder Brom;
$R^5$ in der Formel Ia Wasserstoff, Cyano oder Ethinyl und in der Formel Ib Wasserstoff;
$R^6$ in der Formel Ia Methyl und in der Formel Ib Methyl, Fluor und Chlor.

Ferner betrifft die Erfindung ein Verfahren zur Herstellung der Cyclopropancarbonsäureester I, Schädlingsbekämpfungsmittel, die diese Ester als Wirkstoffe enthalten, sowie Verfahren zur Bekämpfung von Schädlingen mit diesen Wirkstoffen.

Es ist bereits bekannt, daß bestimmte Ester der 3-(2-Phenylethenyl)-2,2-dimethylcyclopropancarbonsäure und Alkoholen die eine Phenoxybenzyl- oderPhenylbenzylstruktur aufweisen insektizide Eigenschaften besitzen (vgl. z.B. US 4 536 591, EP 49 977, US 4 332 815, EP 143 152, DE 27 30 515, DE 29 20 947 und EP 03336).

Die insektizide Wirkung dieser Ester ist jedoch unter bestimmten Bedingungen, z.B. niedrigen Aufwandmengen oder Wirkung an bestimmten Schädlingen, in den meisten Fällen unbefriedigend. Demgemäß bestand die Aufgabe, substituierte Cyclopropancarbonsäurebenzylester mit verbesserter Wirkung bereitzustellen.

Demgemäß wurden die eingangs definierten 2-Phenyl-alk(en)-1-yl-cyclopropancarbonsäurebenzylester Ia und Ib gefunden. Schädlingsbekämpfungsmittel, enthaltend diesen Wirkstoff, weisen eine starke insektizide und akarizide Wirkung auf.

Die Ester der allgemeinen Formeln Ia und Ib können durch Umsetzung von Cyclopropancarbonsäuren IIa oder Derivaten dieser Säuren wie Säurechloriden, Säureanhydriden o.ä., in denen R8 für eine an sich übliche Abgangsgruppe wie Halogen steht oder den Rest OM darstellt, wobei M für ein Alkali- oder Erdalkalimetall wie Natrium, Kalium, Lithium, Calcium oder Magnesium steht, mit Benzylalkoholen der allgemeinen Formel IIIa oder Derivaten hiervon, in denen R9 für eine an sich übliche Abgangsgruppe steht oder den Rest OM, wie vorstehend definiert, darstellt, in an sich bekannter Weise gewonnen werden. Übliche Abgangsgruppen sind z.B. Chlor, Brom, Jod, O-Mesyl, O-Tosyl oder ein fluorierter Alkyl- oder Arylsulfonsäurerest.

Die Umsetzung ausgehend von Cyclopropancarbonsäure IIa' bzw. IIb' ($R^8$ = OH) und dem Benzylalkohol IIIa ($R^9$ = OH) ist in folgendem Reaktionsschema dargestellt:

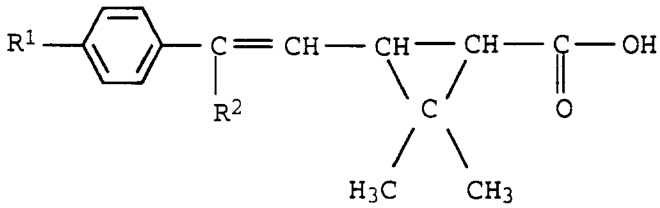

bzw.

IIa'

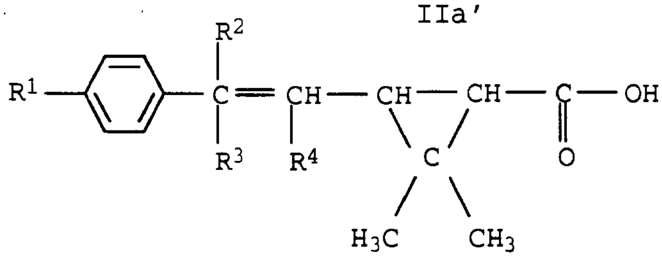

IIb'

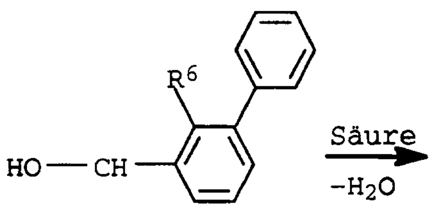


Ia bzw. Ib

III'

Die Veresterung kann in an sich bekannter Weise durch Zusatz von Katalysatoren wie Schwefelsäure, Halogenwasserstoff, Sulfonsäure, sauren Ionenaustauschern beschleunigt und das Veresterungsgleichgewicht im gewünschten Sinne verschoben werden, indem man dem Reaktionsgemisch das Wasser oder den Ester I entzieht, z.B. durch azeotrope Destillation oder durch Bindung des Wassers an Schwefel- oder Halogenwasserstoffsäure.

Ebensogut können die entsprechenden Säurechloride mit den Alkoholen der Formel IIIa in Gegenwart eines Säureakzeptors umgesetzt werden (vgl. Houben-Weyl, Methoden der org. Chemie, Band VIII, S. 541 ff, Georg-Thieme-Verlag, Stuttgart 1952).

Als Säurebindemittel eignen sich die üblichen basischen Mittel, insbesondere aliphatische, aromatische und heterocyclische Amine, z.B. Triethylamin, Dimethylamin, Piperidin, Dimethylanilin, Dimethylbenzylamin, Pyridin und 2-Picolin.

Die Umsetzung kann in einem Lösungs- oder Verdünnungsmittel ausgeführt werden. Hierzu sind die angeführten Säureakzeptoren selbst oder beispielsweise folgende Lösungs- oder Verdünnungsmittel oder Gemische derselben geeignet:
Aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlorethan, Chlorbenzol; Ether wie Diethyl- und Di-n-butylether, Methyl-tert.-butylether,Tetrahydrofuran, Dioxan; Ketone, beispielsweise Aceton, Methylethylketon, Methylisopropylketon; ferner Nitrile wie Acetonitril und Propionitril.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung verläuft oberhalb von 0 °C mit ausreichender Geschwindigkeit. Da sie meist unter Wärmeentwicklung verläuft, kann es von Vorteil sein, eine Kühlmöglichkeit vorzusehen.

In einigen Fällen ist es sinnvoll und vorteilhaft die Verbindungen der Formel IIa insbesondere dann, wenn $R^5$ die Bedeutung einer Cyanogruppe hat, in situ zu verestern.

So erhält man 2-Phenyl-alk(en)-1-yl-cyclopropancarbonsäurebenzylester der Formel Ia, in denen $R^5$ Cyano bedeutet dadurch, daß man Cyclopropancarbonsäuren der Formel IIa, in der $R^8$ für Hydroxy steht und $R^1$ und $R^2$ die vorstehend genannten Bedeutungen haben, mit substituiertem 3-Phenylbenzaldehyd der allgemeinen Formel IV

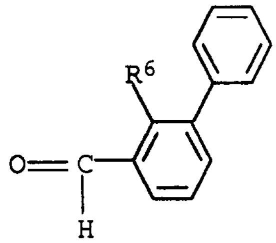

IV

in der $R^6$ die vorstehend angeführte Bedeutung hat, in Gegenwart eines Alkali- oder Erdalkalicyanids bei Temperaturen von -20 °C bis +150 °C gegebenenfalls in Gegenwart eines Lösungsmittels, eines Katalysators, eines Säurebindungsmittels oder unter Einwirkung von Ultraschall umsetzt.

Die erfindungsgemäßen Ester können außerdem noch nach praktisch allen bekannten Verfahren der Estersynthese hergestellt werden, beispielsweise durch Umsetzung von entsprechenden Säureanhydriden mit den Alkoholen der Formel IIIa, durch Umsetzung von entsprechenden Carbonsäure-Salzen mit Derivaten der Alkohole der Formel IIIa oder durch Umesterung (vgl. Houben-Weyl a.a.O., S. 508-628).

Es versteht sich, daß die Verbindungen der Formeln Ia und Ib in jedem Falle in Form von einheitlichen Diastereomeren, mindestens einem Paar optischer Antipoden, in vielen Fällen auch in Form von Diastereomeren-Vielfachen vorkommen und als Wirkstoffe angewandt werden können, die je nach den Ausgangsstoffen und den Umsetzungsbedingungen in einheitlicher Form oder als Gemische auftreten. Ihre biologische Wirkung ist in Einzelfällen von ihrer sterischen Konfiguration abhängig.

Einzelne Stereoisomere der Carbonsäureester nach Anspruch 1 können dadurch erhalten werden, daß man stereoisomerenreine Ausgangsverbindungen einsetzt oder erhaltene Produktgemische nach bekannten Verfahren, wie präp. DC, SC, MPLC oder HPLC mit Hilfe geeigneter Absorbtionsmittel wie Kieselgel oder Aluminiumoxid oder anderer handelsüblicher Absorbtionsmittel und geeigneten Lösungsmitteln, die einzeln oder als Gemisch verwendet werden, beispielweise Petrolether, Pentan, Hexan, Cyclohexan, Diethylether, Methyl- tert.-butylether, Diisopropylether, Toluol, Benzol, Aceton, Methanol, Ethanol, Essigester, Methylenchlorid, Chloroform oder Acetonitril, in ihre sterisch einheitlichen Bestandteile auftrennt.

Verfahren zur Herstellung der eingesetzten Pyrethroidsäuren und ihrer Derivate sind beispielsweise in Wegler, Chemie der Pflanzenschutz- und Schädlingsbekämpfungsmittel, Band 7, S. 57-66 und 137-172, Springer Verlag, Berlin, Heidelberg, New York 1981; Angew. Chem. Int. Ed. Engl. 20, 703-722 (1981); DE 2 738 150; DE 2 920 947; DE 2 916 357; EP 0 095 047 beschrieben.

Verfahren zur Herstellung der eingesetzten Alkohole der allgemeinen Formel III, wenn $R^5$ = Wasserstoff und $R^9$ = OH oder Brom ist, sind beispielsweise in Pest. Sci. 14, 560-570, 1983 beschrieben.

Verbindungen der allgemeinen Formel IV können aus Verbindungen der allgemeinen Formel III, in der $R^9$ OH bedeutet, durch Oxidation nach literatur bekannten Methoden (Houben-Weyl, Methoden der Org. Chemie, Band E 3, S. 275-299) hergestellt werden. Ebenso können diese aus den Verbindungen der allgemeinen Formel III, in der $R^9$ Chlor bzw. Brom oder $OSO_2$-p-Toluol bedeutet, nach literaturbekannten Methoden (Houben-Weyl, Methoden der Org. Chemie, Band E 3, S. 248-265) hergestellt werden.

Außerdem lassen sich die Benzylhalogenide der Formel III ($R^9$ = Cl, Br) in Benzylalkohole der Formel III ($R^9$ = OH) nach literaturbekannten Methoden (Houben-Weyl, Methoden der Org. Chemie, Band 6/1a/1, S. 174-223) umwandeln und anschließend in Verbindungen der Formel IV überführen.

Verbindungen der allgemeinen Formel III, in der $R^5$ Alkyl, Alkenyl, Alkinyl und $R^9$ OH bedeuten, lassen sich aus Verbindungen der allgemeinen Formel IV durch metallorganische Reaktionen nach literaturbekannten Methoden (Houben-Weyl, Methoden der Org. Chemie, Band 6/1a/2) herstellen.

Die Herstellung der erfindungsgemäßen Verbindungen erfolgt gemäß den Beschreibungen und der folgenden Beispiele bzw. durch entsprechende Abwandlungen.

Die Cyclopropancarbonsäurebenzylester der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmitteleingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Ortiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma guadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Zur Klasse der Arachnoidea gehören Spinnentiere (Acarina) beispielsweise Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins meghini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfit-ablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1.1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 1.2 werden mit einer Mischung aus 92 Gew.-Teilen pulverförmigem Kieselsäuregel und 8 Gew.- Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III.10 Gew.-Teile der Verbindung Nr. 1.5 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecyl-benzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 1.7 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 1.1 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 10, vorzugsweise 0,05 bis 2 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiele

A) trans,Z-3- 2-Chlor-2-(4-chlorphenyl)-ethenyl -2,2-dimethylcyclopropylcarbonsäurechlorid

57 g (0,2 mol) der entsprechenden Carbonsäure werden in 400 ml Toluol (trocken) gelöst und mit 174 g (0,22 mol) Pyridin (trocken) versetzt. Bei 0-5 °C werden 59,5 g (0,5 mol) Thionylchlorid zugetropft und anschließend bei Raumtemperatur ca. 24 h nachgerührt. Ein entstandener Niederschlag wird abgesaugt und das Filtrat im Vakuum eingeengt. Das erhaltene Öl wird in Methyl-tert.-butylether aufgenommen, auf 0 °C abgekühlt und ein entstandener Niederschlag abgesaugt. Das Filtrat wird im Vakuum eingeengt und das erhaltene Öl in Pentan/Methyl-tert.-butylether (2/1) gelöst, auf 0 °C abgekühlt und ein eventuell entstandener Niederschlag abgesaugt. Das Filtrat wird eingeengt. Das erhaltene Öl, 60 g = 98,8 % d. Th., ist ausreichend rein um für die Veresterung verwendet zu werden.

250 Mhz $^1H$-NMR-Spektrum in $CDCl_3$:

$\delta$ [ppm] =

s 1.33 (3H); s 1.41 (3H); d 2.28 J = 6.7 Hz (1H); dd 2.74 J = 8.3 u. 6.7 Hz (1H); d 5.86 J = 8.3 Hz (1H); d (br) 7.31 J = 8 Hz (2H); d (br) 7.5 J = 8 Hz (2H)

B) trans,Z-2'-Methyl-3'-phenylbenzyl-3- 2-chlor-2-(4-chlorphenyl)ethenyl -2,2-dimethylcyclopropancarboxylat

1,98 g (0,01 mol) 2-Methyl-3-phenylbenzylalkohol werden in 20 ml Toluol (trocken) mit 0,87 g (0,011 mol) Pyridin (trocken) versetzt. Dazu wird bei Raumtemperatur 3,035 g (0,01 mol) des unter A erhaltenen Säurechlorids, gelöst in 10 ml Toluol (trocken), getropft. Es wird ca. 18 h bei Raumtemperatur nachgerührt. Die Reaktionsmischung wird mit ca. 100 ml Wasser versetzt, die organische Phase wird abgetrennt und die wäßrige Phase mehrfach gründlich mit Methyl-tert.-butylether extrahiert. Die vereinigten organischen Phasen werden nacheinander mit ca. 2n Salzsäure, Wasser und gesättigter Natriumchloridlösung gewaschen, anschließend wird über Natriumsulfat getrocknet. Nach entfernen des Lösungsmittels im Vakuum werden 4,2 g eines gelben zähen Öls erhalten. Nach säulenchromatographischer Reinigung an Kieselgel (230-400 mesh) 1:30 mit Toluol:Cyclohexan 1:2 verbleiben 3,9 g = 84 % d. Th. eines leicht gelben Öls.

250 MHz $^1H$-NMR-Spektrum in $CDCl_3$:

$\delta$ [ppm] =

s 1.25 (3H); s 1.39 (3H); d 1.78 J = 6.7 Hz (1H); s 2.26 (3H); dd 2.59 J = 8.3 u. 6.7 Hz (1H); s 2.5 (2H); d 5.84 J = 8.3 Hz (1H); m 7.2-7.52 (12H).

Nach den beschriebenen Verfahren wurden die in der folgenden Tabelle 1 genannten erfindungsgemäßen Ester der allgemeinen Formel Ia ($R^3 + R^4 = \pi$-Bindung) sowie in Tabelle 2 Ester der allgemeinen Formel Ib ($R^3$,$R^4$ = Halogen) hergestellt, die durch die Angabe physikalischer Daten näher charakterisiert sind; die übrigen Verbindungen der Tabellen können unter Verwendung entsprechender Ausgangsstoffe analog erhalten werden.

Tabelle 1: Verbindungen der Struktur Ia

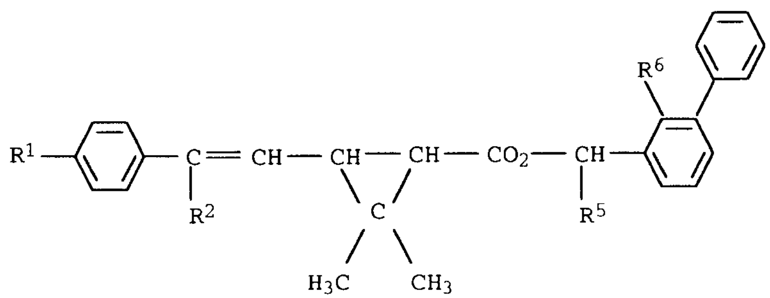

Ia

| Verbindung Nr. | $R^1$ | $R^2$ | $R^5$ | $R^6$ | Konfiguration | physikalische Daten $^1H$-NMR/δ [ppm] $CDCl_3$ |
|---|---|---|---|---|---|---|
| 1.2 | $CF_3$ | Cl | H | $CH_3$ | trans/Z | s 1.25 (3H); s 1.40 (3H); d 1.80 J=6.7Hz (1H); s 2.25 (3H); dd 2.6 J=8.3 u. 6.7Hz (1H); s 5.26 (2H); d 5.97 J=8.3Hz; m 7.2–7.48 (8H); m 7.53–7.72 (4H). |

Tabelle 1: Fortsetzung

| Verbindung Nr. | $R^1$ | $R^2$ | $R^5$ | $R^6$ | Konfiguration | physikalische Daten $^1H$-NMR/δ [PPM] $CDCl_3$ |
|---|---|---|---|---|---|---|
| 1.3 | $CF_3$ | Cl | H | $CH_3$ | trans/E | s 1.23 (3H); s 1.26 (3H); d 1.70 J=6.7Hz (1H); dd 2.12 J=8.8 u. 6.7Hz (1H); s 2.23 (3H); s 5.2 (2H); d 5.83 J=8.8Hz (1H); m 7.15–7.26 (8H); m 7.51–7.68 (4H). |
| 1.4 | $CF_3$ | Cl | H | $CH_3$ | | |
| 1.5 | $CF_3$ | Cl | H | $CH_3$ | cis/E | s 1.16 (3H); s 1.37 (3H); m 1.73–1.86 (2H); s 2.24 (3H); s 5.23 (2H); dd 6.48 J=7.5 u. 2Hz (1H); m 7.1–7.46 (8H); m 7.5–7.7 (4H). |
| 1.6 | $CF_3$ | Cl | CN | $CH_3$ | trans/Z | A: s 1.26 (3H); s 1.36 (3H); d 1.83 J=4.2Hz (1H); s 2.3 (3H); dd 2.68 J=8.3 u. 4.2Hz (1H); d 5.96 J=8.3Hz (1H); s 6.66 (1H); B: s 1.33 (3H); s 1.44 (3H); d 1.8 J=4.2Hz (1H); s 2.33 (3H); dd 2.62 J=8.3 u. 4.2Hz (1H); d 5.95 J=8.3Hz (1H); s 6.68 (1H); A u. B: m 7.21–7.5 (8H); m 7.53– 7.72 (4H). |

Tabelle 1: Fortsetzung

| Verbindung Nr. | $R^1$ | $R^2$ | $R^5$ | $R^6$ | Konfiguration | physikalische Daten $^1H$-NMR/$\delta$[PPM]$CDCl_3$ |
|---|---|---|---|---|---|---|
| 1.7 | $CF_3$ | Cl | CN | $CH_3$ | trans/E | A: s 1.2 (3H); s 1.26 (3H); d 1.7 J=6.7Hz (1H); dd 2.18 J=9.1 u. 6.7Hz (1H); s 2.23 (3H); d 5.83 J=9.1Hz (1H); s 6.56 (1H);<br>B: s 1.28 (3H); s 1.31 (3H); d 1.73 J=6.7Hz (1H); dd 2.1 J=9.1 u. 6.7Hz (1H); s 2.27 (3H); d 5.79 J=9.1Hz (1H); s 6.6 (1H);<br>A u. B: m 7.2–7.7 (12H). |
| 1.8 | $CF_3$ | Cl | CN | $CH_3$ | cis/Z | |
| 1.9 | $CF_3$ | Cl | CN | $CH_3$ | cis/E | |
| 1.10 | $CF_3$ | Cl | C≡CH | $CH_3$ | trans/Z | |
| 1.11 | $CF_3$ | Cl | C≡CH | $CH_3$ | trans/E | |
| 1.12 | $CF_3$ | Cl | C≡CH | $CH_3$ | cis/Z | |
| 1.13 | $CF_3$ | Br | C≡CH | $CH_3$ | cis/E | |
| 1.14 | $CF_3$ | Br | H | $CH_3$ | | |
| 1.15 | $CH_3$ | Cl | H | $CH_3$ | | |

Tabelle 1: Fortsetzung

| Verbindung Nr. | $R^1$ | $R^2$ | $R^5$ | $R^6$ | Konfiguration | physikalische Daten $^1H$-NMR/$\delta$[PPM]$CDCl_3$ |
|---|---|---|---|---|---|---|
| 1.16 | $CH_3$ | Cl | CN | $CH_3$ | | |
| 1.17 | $CH_3$ | Cl | C≡CH | $CH_3$ | | |
| 1.18 | $C_4H_9$ | Cl | H | $CH_3$ | | |
| 1.19 | $C_4H_9$ | Cl | C≡CH | $CH_3$ | | |
| 1.20 | $C_4H_9$ | Cl | CN | $CH_3$ | | |
| 1.21 | $CH_3$ | Br | H | $CH_3$ | | |
| 1.22 | $C_4H_9$ | Br | H | $CH_3$ | | |
| 1.23 | $OCH_3$ | Cl | H | $CH_3$ | | |
| 1.24 | $OCH_3$ | Cl | CN | $CH_3$ | | |
| 1.25 | $OCH_3$ | Cl | C≡CH | $CH_3$ | | |
| 1.26 | $OCH_3$ | Br | H | $CH_3$ | | |
| 1.27 | $OCF_3$ | Cl | H | $CH_3$ | | |
| 1.28 | $OCF_3$ | Br | H | $CH_3$ | | |
| 1.29 | $OCF_3$ | Cl | CN | $CH_3$ | | |
| 1.30 | $OCF_3$ | Cl | C≡CH | $CH_3$ | | |
| 1.31 | F | Cl | H | $CH_3$ | | |
| 1.32 | F | Br | H | $CH_3$ | | |
| 1.33 | F | Cl | CN | $CH_3$ | | |

Tabelle 1: Fortsetzung

| Verbindung Nr. | $R^1$ | $R^2$ | $R^5$ | $R^6$ | Konfiguration | physikalische Daten $^1H$-NMR/$\delta$[PPM]$CDCl_3$ |
|---|---|---|---|---|---|---|
| 1.34 | F | Cl | $C \equiv CH$ | $CH_3$ | | |
| 1.35 | Cl | Cl | H | $CH_3$ | | |
| 1.36 | Cl | Br | H | $CH_3$ | | |
| 1.37 | Cl | Cl | CN | $CH_3$ | | |
| 1.38 | Cl | Cl | $C \equiv CH$ | $CH_3$ | | |

Tabelle 2: Verbindungen der Struktur Ib

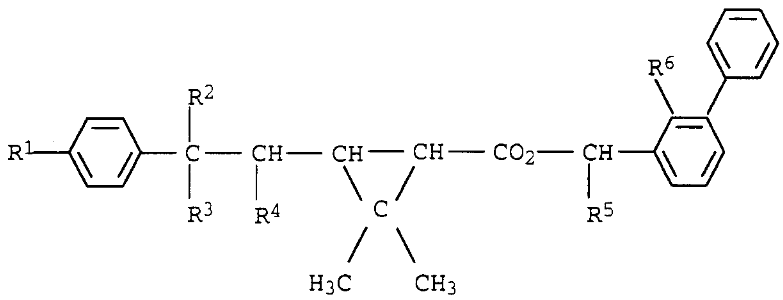

Ib

| Verbindung Nr. | $R^1$ | $R^2$ | $R^3$ | $R^4$ | $R^5$ | $R^6$ |
|---|---|---|---|---|---|---|
| 2.1 | $CF_3$ | Cl | Br | Br | H | $CH_3$ |
| 2.2 | $CF_3$ | Cl | Cl | Cl | H | $CH_3$ |
| 2.3 | $CF_3$ | Br | Br | Br | H | $CH_3$ |
| 2.4 | $CF_3$ | Br | Cl | Cl | H | $CH_3$ |
| 2.5 | Cl | Cl | Br | Br | H | $CH_3$ |
| 2.6 | Cl | Br | Br | Br | H | $CH_3$ |
| 2.7 | Cl | Cl | Cl | Cl | H | $CH_3$ |
| 2.8 | Cl | Br | Cl | Cl | H | $CH_3$ |
| 2.9 | F | Cl | Br | Br | H | $CH_3$ |
| 2.10 | F | Br | Br | Br | H | $CH_3$ |
| 2.11 | F | Cl | Cl | Cl | H | $CH_3$ |
| 2.12 | F | Br | Cl | Cl | H | $CH_3$ |
| 2.13 | $OCH_3$ | Cl | Br | Br | H | $CH_3$ |
| 2.14 | $OCH_3$ | Br | Br | Br | H | $CH_3$ |
| 2.15 | $OCH_3$ | Cl | Cl | Cl | H | $CH_3$ |
| 2.16 | $OCH_3$ | Br | Cl | Cl | H | $CH_3$ |
| 2.17 | $OCF_3$ | Cl | Br | Br | H | $CH_3$ |
| 2.18 | $OCF_3$ | Br | Br | Br | H | $CH_3$ |
| 2.19 | $OCF_3$ | Cl | Cl | Cl | H | $CH_3$ |
| 2.20 | $OCF_3$ | Br | Cl | Cl | H | $CH_3$ |
| 2.21 | $CH_3$ | Cl | Br | Br | H | $CH_3$ |
| 2.22 | $CH_3$ | Br | Br | Br | H | $CH_3$ |
| 2.23 | $CH_3$ | Cl | Cl | Cl | H | $CH_3$ |
| 2.24 | $CH_3$ | Br | Cl | Cl | H | $CH_3$ |
| 2.25 | $t-C_4H_9$ | Cl | Br | Br | H | $CH_3$ |

Tabelle 2: Fortsetzung

| Verbindung Nr. | $R^1$ | $R^2$ | $R^3$ | $R^4$ | $R^5$ | $R^6$ |
|---|---|---|---|---|---|---|
| 2.26 | $t-C_4H_9$ | Br | Br | Br | H | $CH_3$ |
| 2.27 | $t-C_4H_9$ | Cl | Cl | Cl | H | $CH_3$ |
| 2.28 | $t-C_4H_9$ | Br | Cl | Cl | H | $CH_3$ |
| 2.29 | $CF_3$ | Cl | Br | Br | H | Cl |
| 2.30 | $CF_3$ | Cl | Cl | Cl | H | Cl |
| 2.31 | $CF_3$ | Br | Br | Br | H | Cl |
| 2.32 | $CF_3$ | Br | Cl | Cl | H | Cl |
| 2.33 | Cl | Cl | Br | Br | H | Cl |
| 2.34 | Cl | Br | Br | Br | H | Cl |
| 2.35 | Cl | Cl | Cl | Cl | H | Cl |
| 2.36 | Cl | Br | Cl | Cl | H | Cl |
| 2.37 | F | Cl | Br | Br | H | Cl |
| 2.38 | F | Br | Br | Br | H | Cl |
| 2.39 | F | Cl | Cl | Cl | H | Cl |
| 2.40 | F | Br | Cl | Cl | H | Cl |
| 2.41 | $OCH_3$ | Cl | Br | Br | H | Cl |
| 2.42 | $OCH_3$ | Br | Br | Br | H | Cl |
| 2.43 | $OCH_3$ | Cl | Cl | Cl | H | Cl |
| 2.44 | $OCH_3$ | Br | Cl | Cl | H | Cl |
| 2.45 | $OCF_3$ | Cl | Br | Br | H | Cl |
| 2.46 | $OCF_3$ | Br | Br | Br | H | Cl |
| 2.47 | $OCF_3$ | Cl | Cl | Cl | H | Cl |
| 2.48 | $OCF_3$ | Br | Cl | Cl | H | Cl |
| 2.49 | $CH_3$ | Cl | Br | Br | H | Cl |
| 2.50 | $CH_3$ | Br | Br | Br | H | Cl |
| 2.51 | $CH_3$ | Cl | Cl | Cl | H | Cl |
| 2.52 | $CH_3$ | Br | Cl | Cl | H | Cl |
| 2.53 | $t-C_4H_9$ | Cl | Br | Br | H | Cl |
| 2.54 | $t-C_4H_9$ | Br | Br | Br | H | Cl |
| 2.55 | $t-C_4H_9$ | Cl | Cl | Cl | H | Cl |
| 2.56 | $t-C_4H_9$ | Br | Cl | Cl | H | Cl |
| 2.57 | $CF_3$ | Cl | Br | Br | H | F |
| 2.58 | $CF_3$ | Cl | Cl | Cl | H | F |
| 2.59 | $CF_3$ | Br | Br | Br | H | F |
| 2.60 | $CF_3$ | Br | Cl | Cl | H | F |
| 2.61 | Cl | Cl | Br | Br | H | F |

Tabelle 2: Fortsetzung

| Verbindung Nr. | $R^1$ | $R^2$ | $R^3$ | $R^4$ | $R^5$ | $R^6$ |
|---|---|---|---|---|---|---|
| 2.62 | Cl | Br | Br | Br | H | F |
| 2.63 | Cl | Cl | Cl | Cl | H | F |
| 2.64 | Cl | Br | Cl | Cl | H | F |
| 2.65 | F | Cl | Br | Br | H | F |
| 2.66 | F | Br | Br | Br | H | F |
| 2.67 | F | Cl | Cl | Cl | H | F |
| 2.68 | F | Br | Cl | Cl | H | F |
| 2.69 | $OCH_3$ | Cl | Br | Br | H | F |
| 2.70 | $OCH_3$ | Br | Br | Br | H | F |
| 2.71 | $OCH_3$ | Cl | Cl | Cl | H | F |
| 2.72 | $OCH_3$ | Br | Cl | Cl | H | F |
| 2.73 | $OCF_3$ | Cl | Br | Br | H | F |
| 2.74 | $OCF_3$ | Br | Br | Br | H | F |
| 2.75 | $OCF_3$ | Cl | Cl | Cl | H | F |
| 2.76 | $OCF_3$ | Br | Cl | Cl | H | F |
| 2.77 | $CH_3$ | Cl | Br | Br | H | F |
| 2.78 | $CH_3$ | Br | Br | Br | H | F |
| 2.79 | $CH_3$ | Cl | Cl | Cl | H | F |
| 2.80 | $CH_3$ | Br | Cl | Cl | H | F |
| 2.81 | t-$C_4H_9$ | Cl | Br | Br | H | F |
| 2.82 | t-$C_4H_9$ | Br | Br | Br | H | F |
| 2.83 | t-$C_4H_9$ | Cl | Cl | Cl | H | F |
| 2.84 | t-$C_4H_9$ | Br | Cl | Cl | H | F |

Anwendungsbeispiele

In den folgenden Beispielen wurde die Verbindung Nr. 1.1 im Vergleich zu den aus dem Stand der Technik bekannten Verbindungen I und II getestet.

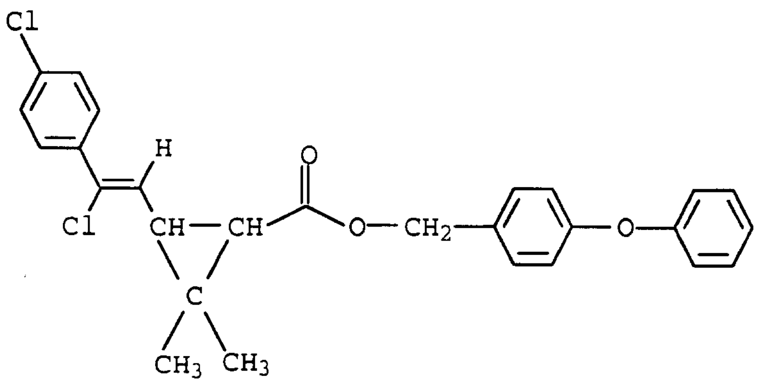

I

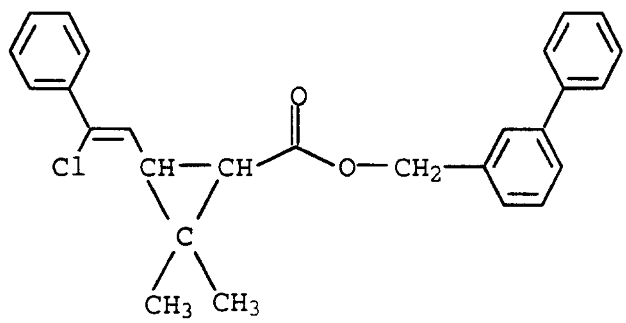

II

Die Reinheit der Substanzen lag bei > 95 %. Die Konzentrationen, bei denen die untersuchten Verbindungen eine 100 %ige Mortalität bewirken, sind die jeweiligen Minimalkonzentrationen. Bei jeder Konzentration wurden mindestens zwei Versuche durchgeführt und ein Mittelwert gebildet.

Der Wirkstoff wurde entweder in Form einer 0,1 %igen acetonischen Lösung verwendet oder in Form eines 10 %igen Emulsionskonzentrates, das durch Emulgieren des Wirkstoffs in einer Mischung, enthaltend 70 Gew.% Cyclohexanon, 20 Gew.% Nekanil® LN (= Lutensol AP6, Netzmittel mit Emulgier- und Dispergierwirkung auf Basis ethorylierter Alkylphenole) und 10 Gew.% Emulphor® EL (Emulgator auf Basis ethoxylierter Fettalkohole), erhalten wurde. Die in den Beispielen angegebenen Konzentrationen wurden durch Verdünnen des formulierten Wirkstoffes mit Wasser erhalten.

Kontaktwirkung auf Blatta orientalis (Schabe)

Der Boden eines 1 l-Glases wird mit der acetonischen Lösung des Wirkstoffes behandelt. Nach Verdunsten des Lösungsmittels setzt man je Glas 5 adulte Schaben. Die Mortalitätsrate wird nach 48 Stunden bestimmt.

In diesem Versuch wies die Verbindung Nr. 1.1 eine 10mal höhere Aktivität auf als die Vergleichssubstanz I. Die Vergleichssubstanz II war bei einer Konzentration von 1 mg unwirksam.

Kontaktwirkung auf Sitophilus granaria (Kornkäfer)

Der Boden von Glaspetrischalen (Ø 10 cm), deren Rand mit FLUON behandelt ist, wird mit 1 cm3 der acetonischen Wirkstofflösung behandelt.

Nach dem Verdunsten des Acetons werden die Schalen mit ca. 50 Kornkäfern besetzt. Nach 4 Stunden werden die Käfer in Pappschälchen (Ø 40 mm, Höhe 10 mm) umgesetzt und in die Petrischale zurückgestellt. Die Wirkung bestimmt man nach 24 Stunden in % Mortalität.

Käfer, die das Pappschälchen nicht mehr verlassen können, gelten als tot bzw. schwer geschädigt.

In diesem Versuch war die Verbindung 1.1 der Vergleichssubstanz überlegen, während die Vergleichssubstanz II bei 10mal höherer Konzentration als die erfindungsgemäße Verbindung unwirksam war.

Kontaktwirkung auf Aedes aegypti (Gelbfiebermücke)

200 ml Leitungswasser in 250 ml Plastikbechern werden mit der Wirkstoffaufbereitung versetzt und daraufhin mit 20 bis 30 Moskitolarven im 3. bis 4. Larvenstadium besetzt. Die Versuchstemperatur beträgt 25'C. Nach 24 Stunden wird die Wirkung ermittelt.

In diesem Versuch war die Verbindung 1.1 den Vergleichssubstanzen weitaus überlegen.

Dauerkontaktwirkung auf Musca domestica (Stubenfliegen)

Beide Teile einer Petrischale von 10 cm Durchmesser werden mit insgesamt 2 ml der acetonischen Wirkstofflösung ausgekleidet. Nach Verdunsten des Lösungsmittels (ca. 30 Minuten) bringt man je 10 Fliegen in die Schalen. Die Mortalität wird nach 4 Stunden festgestellt.

In diesem Versuch war Verbindung Nr. 1.1 den Vergleichssubstanzen I und II überlegen.

Kontaktwirkung auf Prodenia litura

Glaspetrischalen (Ø 10 cm) werden mit der acetonischen Wirkstoffaufbereitung behandelt. Nach Verdunsten des Lösungsmittels werden 5 Raupen im 4. Larvenstadium eingesetzt und die Schalen verschlossen. Nach 4 Stunden wird die Mortalität festgestellt.

In diesem Versuch war die Verbindung 1.1 der Vergleichssubstanz II überlegen. Vergleichssubstanz I zeigte bei höherer Konzentration von 1 mg 0 % Mortalität.

Kontaktwirkung auf Plutella maculipennis (Kohlenschaben-Raupen)

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt. Nach 48 Stunden beurteilt man die Mortalität.

In diesem Versuch zeigte Verbindung 1.1 eine 50mal höhere Aktivität als die Vergleichssubstanz I.

Kontaktwirkung auf Ornithodorus moubata (Zecke)

Verwendet werden junge Zecken, die eine Blutmahlzeit hinter sich haben (Ø 1,5-2 mm). Diese Tiere nimmt man mit Hilfe eines Saugrohres einzeln auf, wobei eine starke Lichtquelle die aktiven Tiere aus den Häutungsresten treibt.

Je 5 Zecken werden in einem Papierbeutel für 5 sec. in die wäßrige Wirkstoffaufbereitung getaucht. Die Beutel hängt man danach frei auf und ermittelt nach 48 Stunden die Wirkung. Dabei werden die Beutel an eine starke Lichtquelle (60 Watt-Birne) gehalten; die lebenden Tiere versuchen auszuweichen und sind leicht zu erkennen.

In diesem Versuch zeigte Verbindung 1.1 eine 20mal höhere Aktivität als die Vergleichssubstanz I und eine 40mal höhere Aktivität als die Vergleichssubstanz II.

## Patentansprüche

**1.** Substituierte 2-Phenyl-alk(en)-1-yl-cyclopropancarbonsäurebenzylester der allgemeinen Formeln Ia und Ib

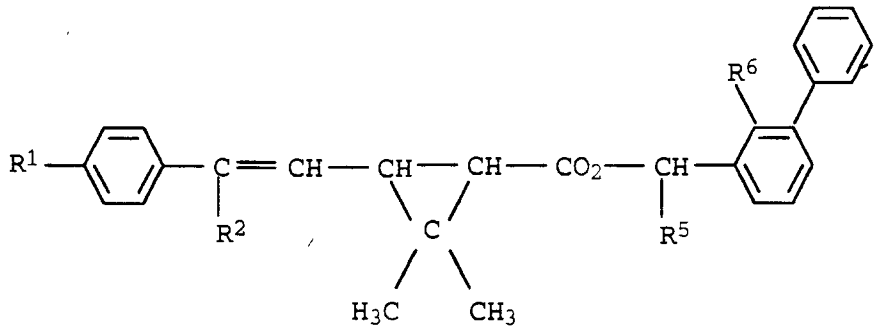

Ia

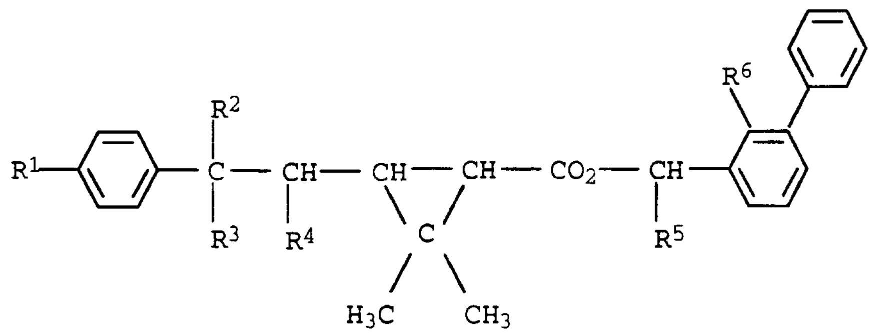

Ib

in denen die Substituenten die folgende Bedeutung haben:

| | |
|---|---|
| $R^1$ | Fluor, Chlor, Methyl, t.-Butyl, Trifluormethyl, Methoxy oder Trifluormethoxy; |
| $R^2$ | Chlor oder Brom; |
| $R^3$ und $R^4$ | gleichzeitig Chlor oder Brom; |
| $R^5$ | in der Formel Ia Wasserstoff, Cyano oder Ethinyl und in der Formel Ib Wasserstoff; |
| $R^6$ | in der Formel Ia Methyl und in der Formel Ib Methyl, Fluor und Chlor. |

**2.** Verfahren zur Herstellung von 2-Phenyl-alk(en)-1-yl-cyclopropancarbonsäurebenzylestern gemäß Anspruch 1, dadurch gekennzeichnet, daß man Cyclopropancarbonsäuren oder -säurederivate der allgemeinen Formel IIa bzw. IIb

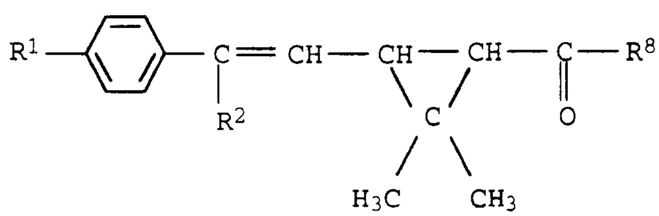

IIa

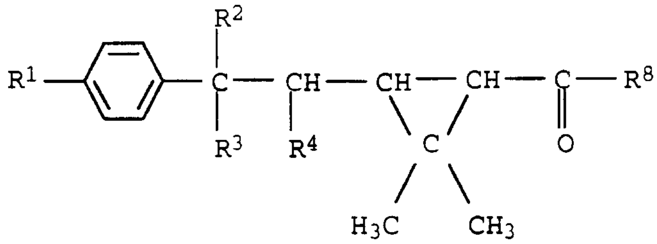

IIb

in der $R^1$ bis $R^4$ die in Anspruch 1 angeführten Bedeutungen haben und $R^8$ für Hydroxy, Chlor, Brom oder OM steht, wobei M ein Alkali- oder Erdalkalimetall bedeutet, mit Biphenylderivaten der allgemeinen Formel III

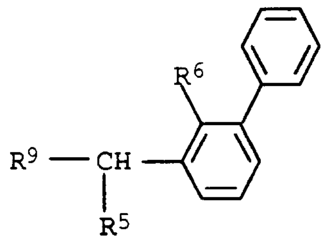

III

in der $R^5$ und $R^6$ die in Anspruch 1 genannten Bedeutungen haben und $R^9$ für Hydroxy oder eine an sich übliche Abgangsgruppe steht, in an sich bekannter Weise umsetzt.

**3.** Verfahren zur Herstellung von 2-Phenyl-alk(en)-1-yl-cyclopropancarbonsäurebenzylestern der Formel Ia gemäß Anspruch 1, in denen $R^5$ Cyano bedeutet, dadurch gekennzeichnet, daß man Cyclopropancarbonsäuren der Formel IIa, in der $R^8$ für Hydroxy steht und $R^1$ und $R^2$ die in Anspruch 1 genannten Bedeutungen haben, mit substituiertem 3-Phenylbenzaldehyd der allgemeinen Formel IV

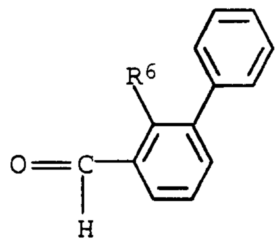

IV

in der $R^6$ die in Anspruch 1 angeführte Bedeutung hat, in Gegenwart eines Alkali- oder Erdalkalicyanids bei Temperaturen von -20 °C bis +150 °C gegebenenfalls in Gegenwart eines Lösungsmittels, eines Katalysators, eines Säurebindungsmittels oder unter Einwirkung von Ultraschall umsetzt.

**4.** Schädlingsbekämpfungsmittel, enthaltend einen substituierten 2-Phenylalk(en)-1-yl-cyclopropancarbonsäurebenzylester gemäß Anspruch 1 sowie feste oder flüssige Trägerstoffe.

**5.** Verfahren zur Bekämpfung von Schädlingen, dadurch gekennzeichnet, daß man einen 2-Phenyl-alk(en)-1-yl-cyclopropancarbonsäurebenzylester Ia oder Ib gemäß Anspruch 1 auf die Schädlinge bzw. deren Lebensraum einwirken läßt.

**6.** Verwendung von 2-Phenyl-alk(en)-1-yl-cyclopropancarbonsäurebenzylbenzylestern gemäß Anspruch 1 zur Bekämpfung von Schädlingen.

**Claims**

1. A substituted benzyl 2-phenyl-1-alk(en)ylcyclopropanecarboxylate of the general formula Ia or Ib

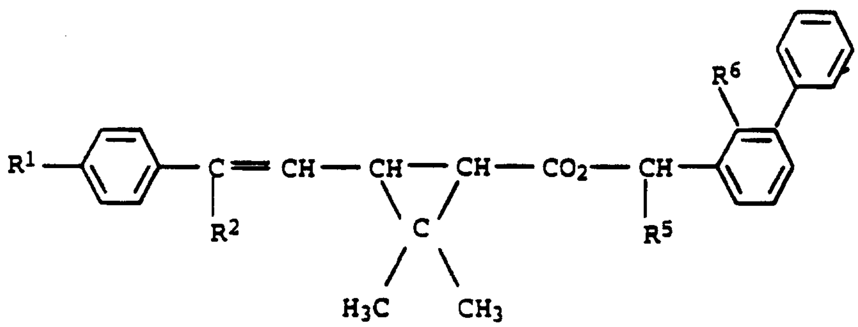

Ia

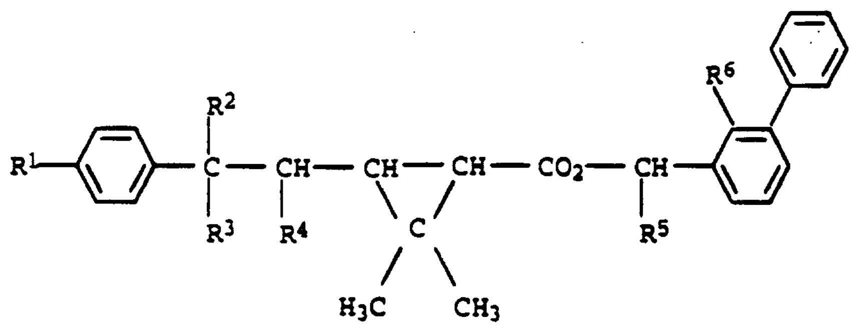

Ib

where

| | |
|---|---|
| $R^1$ | is fluorine, chlorine, methyl t-butyl, trifluoromethyl, methoxy or trifluoromethoxy; |
| $R^2$ | is chlorine or bromine; |
| $R^3$ and $R^4$ | are simultaneously chlorine or bromine; |
| $R^5$ | is hydrogen, cyano or ethynyl in formula Ia and hydrogen in formula Ib; |
| $R^6$ | is methyl in formula Ia and methyl, fluorine and chlorine in formula Ib. |

2. A process for preparing benzyl 2-phenyl-1-alk(en)ylcyclopropanecarboxylatesas claimed in claim 1, which comprises reacting, in a conventional manner, cyclopropanecarboxylic acids or derivatives thereof of the general formula IIa or IIb

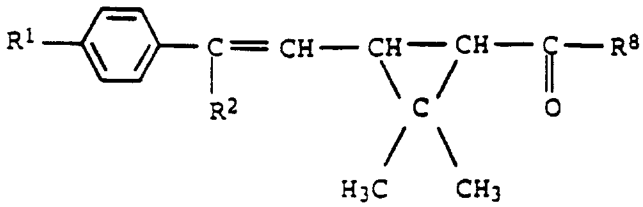

IIa

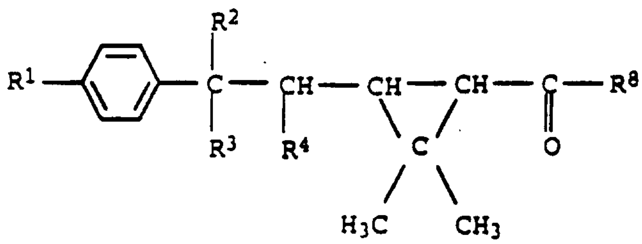

IIb

where $R^1$ to $R^4$ have the meanings stated in claim 1, and $R^8$ is hydroxyl, chlorine, bromine or OM where M is an alkali metal or alkaline earth metal, with biphenyl derivatives of the general formula III

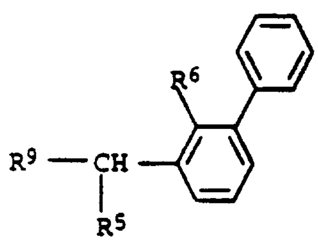

III

where $R^5$ and $R^6$ have the meanings specified in claim 1, and $R^9$ is hydroxyl or a conventional leaving group.

**3.** A process for preparing benzyl 2-phenyl-1-alk(en)ylcyclopropanecarboxylatesof the formula Ia as claimed in claim 1, where $R^5$ is cyano, which comprises reacting cyclopropanecarboxylic acids of the formula IIa where $R^8$ is hydroxyl, and $R^1$ and $R^2$ have the meanings specified in claim 1, with substituted 3-phenylbenzaldehyde of the general formula IV

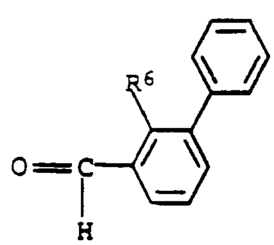

IV

where $R^6$ has the meanings stated in claim 1, in the presence of an alkali metal cyanide or alkaline earth metal cyanide at from -20 °C to +150 °C in the presence or absence of a solvent, of a catalyst, of an acid binder or with exposure to ultrasound.

**4.** A pest control agent containing a substituted benzyl 2-phenyl-1-alk(en)ylcyclopropanecarboxylate as claimed in claim 1 together with solid or liquid carriers.

**5.** A method for controlling pests, which comprises allowing a benzyl 2-phenyl-1-alk(en)-ylcyclopropanecarboxylate Ia or Ib as claimed in claim 1 to act on the pests or their habitat.

6. The use of benzyl 2-phenyl-1-alk(en)ylcyclopropanecarboxylates as claimed in claim 1 for controlling pests.

**Revendications**

1. 2-Phényl-alk(én)-1-yl-cyclopropanecarboxylates de benzyle substitués des formules générales Ia et Ib

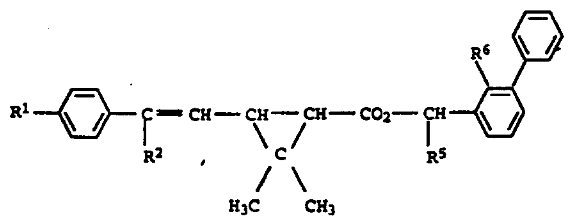

Ia

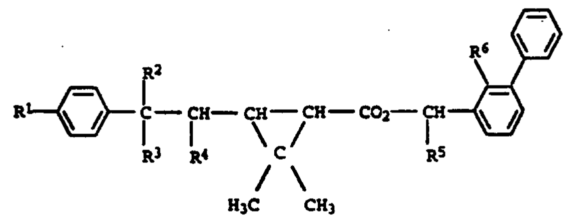

Ib

dans lesquelles les substituants ont les significations suivantes :
$R^1$ fluor, chlore, méthyle, ter-butyle, trifluorométhyle, méthoxy ou trifluorométhoxy;
$R^2$ chlore ou brome;
$R^3$ et $R^4$, simultanément chlore ou brome;
$R^5$ dans la formule Ia, hydrogène, cyano ou éthynyle et dans la formule Ib, hydrogène;
$R^6$ dans la formule Ia, méthyle et dans le formule Ib, méthyle, fluor ou chlore.

2. Procédé de préparation de 2-phényl-alk(én)-1-yl-cyclopropane-carboxylates de benzyle selon la revendication 1, caractérisé par le fait que l'on fait réagir, de manière, en soi, connue, des acides cyclopropanecarboxyliques ou dérivés d'acides cyclopropanecarboxyliques des formules générales IIa ou IIb

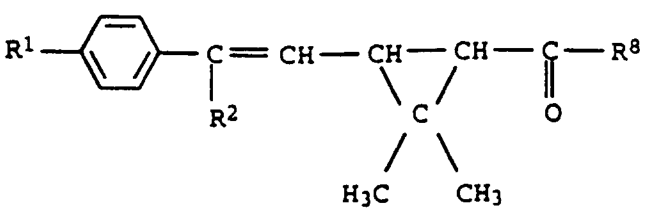

IIa

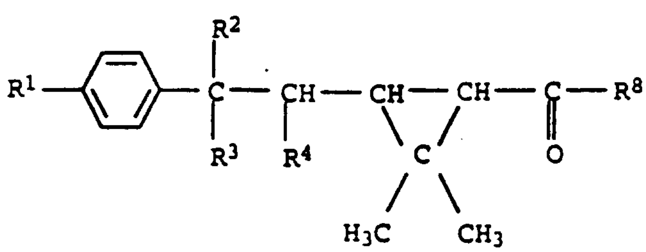

IIb

dans lesquelles $R^1$ à $R^4$ ont les significations données dans la revendication 1 et $R^8$ est mis pour hydroxy, chlore, brome ou OM, M représentant un métal alcalin ou alcalino-terreux, avec les dérivés de biphényle de formule générale III

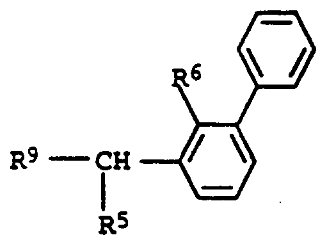

III

dans laquelle $R^5$ et $R^6$ ont les significations données dans la revendication 1 et R9 est mis pour hydroxy ou un groupe éliminable connu en soi.

**3.** Procédé de préparation de 2-phényl-alk(én)-1-yl-cyclopropane-carboxylates de benzyle de formule Ia, selon la revendication 1, dans laquelle $R^5$ représente cyano, caractérisé par le fait que l'on fait réagir en présence d'un cyanure alcalin ou alcalino-terreux, à des températures comprises entre -20 degrés C et + 150 degrés C, éventuellement en présence d'un solvant, d'un catalyseur, d'un liant d'acide ou sous l'action d'ultrasons, des acides cyclopropanecarboxyliques de formule IIa, dans laquelle $R^8$ est mis pour hydroxy, $R^1$ et $R^2$ ayant les significations données dans la revendication 1, avec du 3-phénylbenzaldéhyde substitué, de formule générale IV,

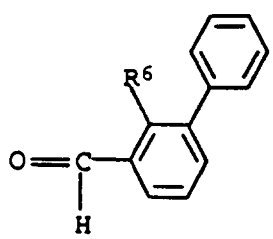

IV

dans laquelle $R^6$ a la signification donnée dans la revendication 1.

**4.** Pesticides, contenant un 2-phénylalk(én)-1-yl-cyclopropanecarboxylate de benzyle substitué selon la revendication 1, ainsi que des supports solides ou liquides.

**5.** Procédé de lutte contre les parasites, caractérisé par le fait que l'on fait agir sur les parasites ou leur biotope, un 2-phényl-alk(én)-1-yl-cyclopropanecarboxylate de benzyle Ia ou Ib selon la revendication 1.

**6.** Utilisation de 2-phényl-alk(èn)-1-cyclopropanecarboxylates de benzyle, selon la revendication 1, pour la lutte contre les parasites.